# EUROPEAN PATENT SPECIFICATION

(11) **EP 0 769 958 B1**
(45) Date of publication and mention of the grant of the patent: **16.04.2003**
(21) Application number: 95925545.6
(22) Date of filing: 10.07.1995
(51) Int. Cl.: A61K 38/04, A61K 38/12, C07K 5/00, C07K 7/00

(54) **INHIBITORS OF LEUKOCYTE ADHESION**
INHIBITOREN DER LEUKOZYTENADHÄSION
INHIBITEURS DE L'ADHESION LEUCOCYTAIRE

(30) Priority: 11.07.1994 US 273055; 06.06.1995 US 467580
(43) Date of publication of application: 02.05.1997
(73) Proprietor: ATHENA NEUROSCIENCES, INC., South San Francisco, CA 94080 (US)
(72) Inventor: THORSETT, Eugene D., Moss Beach, CA 94038 (US); YEDNOCK, Theodore A., Fairfax, CA 94930 (US); PLEISS, Michael A., Fremont, CA 94356 (US)
(74) Representative: Dubois-Chabert, Guy
(86) International application number: US9508516
(87) International publication number: WO96001644

(56) References cited:
- EP-A- 0 364 690
- WO-A-88/07055
- WO-A-94/05310
- WO-A-94/05314
- WO-A-94/25043
- WO-A-95/15973
- T.A. FERGUSON ET AL.: "Two integrin-binding peptides abrogate T cell-mediated immune responses in vivo" PROCEEDINGS OF THE NATIONAL ACADEMY OF SCIENCES OF USA, vol. 88, September 1991 (1991-09), pages 8072-8076, XP002115562 WASHINGTON US

## Description

### BACKGROUND OF THE INVENTION

### Field of the Invention

This invention relates to inhibitors of leukocyte adhesion. In particular, it relates to oligopeptides that block adhesion mediated by VLA-4.

### References

The following publications, patents and patent applications are cited in this application as superscript numbers:
¹ Hemler and Takada, *European Patent Application Publication No.* 330,506, published August 30, 1989
² Elices, et al., *Cell,* 60:577-584 (1990)
³ Springer, *Nature,* 346:425-434 (1990)
⁴ Osborn, *Cell,* 62:3-6 (1990)
⁵ Vedder, et al., *Surgery,* 106:509 (1989)
⁶ Pretolani, et al., *J. Exp. Med.,* 180:795 (1994)
⁷ Abraham, et al., *J. Clin. Invest.,* 93:776 (1994)
⁸ Mulligan, et al., *J. Immunology*, 150:2407 (1993)
⁹ Cybulsky, et al., *Science,* 251:788 (1991)
¹⁰ Li, et al., *Arterioslcer. Thromb*., 13:197 (1993)
¹¹ Sasseville, et al., *Am. J. Path*., 144:27 (1994)
¹² Yang, et al., *Proc. Nat. Acad. Science (USA)*, 90:10494 (1993)
¹³ Burkly, et al., *Diabetes*, 42:529 (1994)
¹⁴ Baron, et al., *J. Clin. Invest.,* 93:1700 (1994)
¹⁵ Hamann, et al., *J. Immunology,* 152:3238 (1994)
¹⁶ Yednock, et al., *Nature*, 356:63 (1992)
¹⁷ Baron, et al., *J. Exp. Med*., 177:57 (1993)
¹⁸ van Dinther-Janssen, et al., *J. Immunology,* 147:4207 (1991)
¹⁹ van Dinther-Janssen, et al., *Annals. Rheumatic Dis.,* 52:672 (1993)
²⁰ Elices, et al., *J. Clin. Invest.,* 93:405 (1994)
²¹ Postigo, et al., *J. Clin. Invest.,* 89:1445 (1991)
²² Paul, et al., *Transpl. Proceed*., 25:813 (1993)
²³ Okarhara, et al., *Can. Res.,* 54:3233 (1994)
²⁴ Paavonen, et al., *Int. J. Can.,* 58:298 (1994)
²⁵ Schadendorf, et al., *J. Path*., 170:429 (1993)
²⁶ Bao, et al., *Diff*., 52:239 (1993)
²⁷ Lauri, et al., *British J. Cancer*, 68:862 (1993)
²⁸ Kawaguchi, et al., *Japanese J. Cancer Res.,* 83:1304 (1992)
²⁹ Sambrook, et al., *Molecular Cloning, A Laboratory Manual,* Cold Spring Harbor Press, Cold Spring Harbor, New York (1982)
³⁰ Matteucci, et al., *J. Am. Chem. Soc*. 103:3185 (1981)
³¹ Robey, et al., *Anal. Biochem.,* 177:373-377 (1989)
³² Robey, et al., U.S. Patent No. 5,066,716, issued November 19, 1991
³³ International Patent Application Publication No. WO 91/05038
³⁴ Verhoef, et al., *Eur. J. Drug Metab. Pharmacokin*., 11:291-302 (1986)
³⁵ Larger, *Science,* 249:1527-1533 (1990)
³⁶ Szoka et al., *Ann. Rev. Biophys. Bioeng*., 9:467 (1980)
³⁷ Papahadjopoulos, et al., U.S. Patent No. 4,235,871, issued November 15, 1980
³⁸ Geho, et al., U.S. Patent No. 4,501,728, issued February 26, 1985
³⁹ Allen, U.S. Patent No. 4,837,028, issued June 6, 1989
⁴⁰ Wayner and Kovach, *J. Cell Biol.,* 116:489-497 (1992)
⁴¹ Vonderheide, et al., *J. Cell Biol.,* 125:215-222 (1994)
⁴² *J. Cell. Biol.,* 117:461-470 (1992)
⁴³ *J. Cell. Biol*., 117:659-670 (1992)

### State of the Art

VLA-4 (also referred to as α4β1 integrin and CD49d/CD29), first identified by Hemler and Takada¹ is a member of the β1 integrin family of cell surface receptors, each of which comprises two subunits, an α chain and a β chain. VLA-4 contains an α4 chain and a β1 chain. There are at least nine β1 integrins, all sharing the same β1 chain and each having a distinct α chain. These nine receptors all bind a different complement of the various cell matrix molecules, such as fibronectin, laminin, and collagen. VLA-4, for example, binds to fibronectin. VLA-4 is unique among β₁ integrins in that it also binds non-matrix molecules that are expressed by endothelial and other cells. These non-matrix molecules include VCAM-1, which is expressed on cytokine-activated human umbilical vein endothelial cells in culture. Distinct epitopes of VLA-4 are responsible for the fibronectin and VCAM-1 binding activities and each activity has been shown to be inhibited independently.²

Intercellular adhesion mediated by VLA-4 and other cell surface receptors is associated with a number of inflammatory responses. At the site of an injury or other inflammatory stimulus, activated vascular endothelial cells express molecules that are adhesive for leukocytes. The mechanics of leukocyte adhesion to endothelial cells involves, in part, the recognition and binding of cell surface receptors on leukocytes to the corresponding cell surface molecules on endothelial cells. Once bound, the leukocytes migrate across the blood vessel wall to enter the injured site and release chemical mediators to combat infection. For reviews of adhesion receptors of the immune system, *see,* for example, Springer³ and Osborn⁴.

Inflammatory brain disorders, such as experimental autoimmune encephalomyelitis (EAE), multiple sclerosis (MS) and meningitis, are examples of central nervous system disorders in which the endothelium/leukocyte adhesion mechanism results in destruction to otherwise healthy brain tissue. Large numbers of leukocytes migrate across the blood brain barrier (BBB) in subjects with these inflammatory diseases. The leukocytes release toxic mediators that cause extensive tissue damage resulting in impaired nerve conduction and paralysis.

In other organ systems, tissue damage also occurs via an adhesion mechanism resulting in migration or activation of leukocytes. For example, it has been shown that the initial insult following myocardial ischemia to heart tissue can be further complicated by leukocyte entry to the injured tissue causing still further insult (Vedder et al.⁵). Other inflammatory conditions mediated by an adhesion mechanism include, by way of example, asthma⁶⁻⁸, Alzheimer's disease, atherosclerosis⁹⁻¹⁰, AIDS dementia¹¹, diabetes¹²⁻¹⁴, inflammatory bowel disease¹⁵, multiple sclerosis¹⁶⁻¹⁷, rheumatoid arthritis¹⁸⁻²¹, tissue transplantation²² and tumor metastasis²³⁻²⁸.

Documents WO-A-94 05314 and WO-A-94 05310 disclose peptide inhibitors of leukocyte adhesion. These peptides inhibit binding of selectins such as P-, E-or L-selectins.

Document EP-A-0 364 690 discloses peptide inhibitors of leukocyte cells surface adhesion, in particular cloning and expression of the alpha-subunit of the Mac-1 receptor molecule through recombinant technology.

Despite these advances in the understanding of leukocyte adhesion, the prior art lacks small, peptidic inhibitors of adhesion useful in the treatment of inflammatory brain diseases and other inflammatory conditions. The present invention addresses these and other needs.

### SUMMARY OF THE INVENTION

This invention provides compositions comprising peptides which, in monomer form, comprise from about 4 to about 13 amino acid residues, preferably from about 6 to about 10, which inhibit cellular adhesion mediated by VLA-4, for example, binding of VCAM-1 to VLA-4. The peptides may be either monomeric or dimeric and comprise peptides having a binding affinity to VLA-4 as expressed by an IC₅₀ of about 50 µM or less (measured as per Example 10 below), which peptides comprise an amino acid sequence YYGN or R-PVSF-R',
where R and R' are independently peptide sequences of from 0 to 7 with the proviso that the sum of amino acid residues in R + R' is no more than 9,
wherein one or more of the amino acid residues in said peptides are optionally D-amino acid residues, further wherein the N-terminus of said peptide is optionally modified by linkage of the amine to a group of the formula R₄-CO- wherein, R₄ is hydrogen, lower alkyl, cycloalkyl, aryl, arylalkyl, or R₅O- where R₅ is lower alkyl, cycloalkyl, aryl and arylalkyl; and
still further wherein the C-terminus of said peptide is optionally modified by linkage of the carbonyl [C(O)] to a group selected from -O-R₆ and 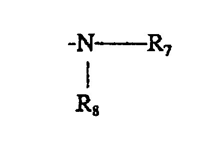 wherein R₆ is lower alkyl, cycloalkyl, aryl or arylalkyl and R₇ and R₈ are the same or different and are hydrogen, lower alkyl, cycloalkyl, aryl, or arylalkyl.

Peptides as defined by the formulas set forth above which have an IC₅₀ of about 50 µM or less (as per Example 6 below) include SEQ ID NOs. 4, 9, 12, 15, 17, 22, 31, 32, 40, 41, 42, 43, 45, 52, 54, 60, 62, 63, 71, 94, 96, 123, 124, 125, 133, 140, 142, 144, 150, and 154.

In some embodiments, the peptides are dimeric. The peptides may, for instance, comprise a C residue at the C-terminus. Preferred peptides include SEQ ID Nos. 71, 123, 125, 140, 144, 150 and 154.

This invention also provides pharmaceutical compositions comprising a pharmaceutically acceptable carrier and a therapeutically effective amount of one or more of the peptides of the invention. The pharmaceutical compositions may be used to inhibit adhesion of leukocytes to endothelial cells, for instance, in treating an inflammatory disease in a patient such as asthma, Alzheimer's disease, atherosclerosis, AIDS dementia, diabetes, inflammatory bowel disease, rheumatoid arthritis, tissue transplantation, tumor metastasis and myocardial ischemia. The compositions can also be administered for the treatment of inflammatory brain disorders, such as multiple sclerosis.

### Definitions

The terms "peptide", "oligopeptide" or "polypeptide" are used to designate a series of amino acids residues or amino acid mimetics connected one to the other typically between the alpha-amino and alpha-carboxy groups of adjacent amino acids. The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification does not destroy the biological activity of the polypeptides as herein described.

An oligopeptide or peptide as used herein refers to a chain of at least about 4 residues, preferably at least about 8. The peptides are usually fewer than about 25 residues, more usually fewer than about 20 and preferably less than about 15. The peptides will typically range between about 8 and 10 residues.

The nomenclature used to describe peptide compounds follows the conventional practice wherein the amino group is presented to the left (the N-terminus) and the carboxyl group to the right (the C-terminus) of each amino acid residue. In the formulas representing selected specific embodiments of the present invention, the amino- and carboxyl-terminal groups, although not specifically shown, are in the form they would assume at physiologic pH values, unless otherwise specified. In the amino acid structures, each residue is generally represented by standard three letter or single letter designations. The L-form of an amino acid residue is represented by a capital single letter or a capital first letter of a three-letter symbol, and the D-form for those amino acids having D-forms is represented by a lower case single letter or a lower case three letter symbol. Glycine has no asymmetric carbon atom and is simply referred to as "Gly" or G.

The term "cyclic" refers to peptides in which the N-tenninal residue is linked to the C-terminal residue either directly or through an intermediate. Examples of links between the two residues include disulfide bonds and thioether linkages as described below.

The term "dimer" refers to peptides in which the C-terminal residue of one oligopeptide is linked to the C-terminal residue of another oligopeptide directly or through an intermediate. An example of a link between the two residues include disulfide bonds as described below.

The term "isolated" or "isolate" refers to material which is substantially or essentially free from components which normally accompany it as found in its native state. Thus, the peptides of this invention do not contain materials normally associated with their *in situ* environment.

The term "residue" refers to an amino acid or amino acid mimetic incorporated in a peptide by an peptide bond or peptide bond mimetic.

The term "alkyl" as used herein means a branched or unbranched, saturated or unsaturated, monovalent or divalent, hydrocarbon radical having from 1 to 20 carbon atoms, including but not limited to lower alkyls of 1-10 carbon atoms such as methyl, ethyl, n-propyl, butyl (i.e., n-butyl, iso-butyl, sec-butyl and t-butyl), n-hexyl, and the like. This definition and the definitions below apply both when the term is used alone and when it is used as part of a compound term, such as "arylalkyl" and the like.

The term "acyl" as used herein refers to an carbonyl radical (CO) to which is appended an alkyl group, especially a lower alkyl group. Examples include radicals such as acetyl, propionyl and the like.

The term "cycloalkyl" as used herein refers to an aliphatic ring having 3 to 7 carbon atoms including, but not limited to, cyclopropyl, cylcobutyl, cyclopentyl, cyclohexyl and cycloheptyl. Preferred cycloalkyls include cyclopropyl, cyclopentyl, and cyclohexyl.

The term "aryl" as used herein refers to a monocyclic or polycyclic carbon ring system having from 6 to 14 carbon atoms including, but not limited to, phenyl, naphthyl, and the like. Preferred aryls include phenyl and naphthyl. Any of the aryl groups described herein may be optionally substituted with halogen atoms (i.e., fluoro, chloro, bromo and iodo), or other groups such as nitro, carboxyl, lower alkyl, lower alkoxy (O-lower alkyl), acyl, cycloalkyl, and the like.

### BRIEF DESCRIPTION OF THE DRAWINGS

Fig. 1 shows the results of experiments measuring the ability of immobilized peptides of the invention to promote adhesion of Jurkat cells in the presence and absence of an anti-VLA-4 antibody.
Fig. 2 shows the results of experiments demonstrating the ability of peptides of the invention to block adhesion of soluble VCAM-1-IgG conjugates to Jurkat cells.

### DESCRIPTION OF THE PREFERRED EMBODIMENTS

This invention provides oligopeptides which comprise adhesion signal sequences recognized by VLA-4. Thus, the peptides can be used to block VLA-4-mediated adhesion and inhibit immunopathologies associated with this adhesion.

The invention is based, in part, upon sequence analysis of 5 monoclonal antibodies against α4 integrin that inhibit VLA-4 binding to VCAM-1. This analysis revealed a short consensus stretch of amino acids within the heavy chain CDR3 of these antibodies. Three of the anti-α4 antibodies contain YGN...Y, while the fourth contains FGN...Y, (Y is typically considered to be a conservative amino acid substitution for F). The sequence of the fifth antibody is completely unrelated. Since the heavy chain CDR3 is the most variable region of an antibody molecule, the chances of obtaining this sequence at random in four separate antibodies is extremely remote. These sequences share homology with sequences within domain 1 of VCAM-1, FGN...Y, as well. In addition, sequences derived from other regions of domain 1 of VCAM-1 also block interaction between VCAM-1 and VLA-4. One example of a known adhesion signal from domain 1 is QIDS (Vonderheide, et al.,⁴¹).

The peptides of the invention thus comprise sequences derived from the sequences of the anti-VLA-4 antibodies noted above or from domains of VCAM-1. These sequences present adhesion signals which allow the peptides to inhibit VLA-4 mediated adhesion *in vivo*, for instance by binding VLA-4 thereby disrupting the binding of VLA-4 to VCAM-1. Preferred adhesion signal sequences are derived from the sequences YYGN, YFGN, FGNE, YGNE or FENE. Peptides incorporating these sequences and their analogs, when appropriately presented, inhibit the binding of protein VCAM-1 to cells that express VLA-4, as demonstrated below.

The affinity of the peptides of the invention for the VLA-4 binding site related to VCAM-1 binding may be determined using the assay described in Example 10, below. The IC₅₀ values of the peptides of the invention, as determined by this assay, are generally below about 600µM, preferably below about 100 µM, and more preferably less than about 50µM. Dimeric peptides of the invention sometimes have better affinity than linear peptides.

The peptides of the invention can be prepared in a wide variety of ways. Because of their relatively short size, the peptides can be synthesized in solution or on a solid support in accordance with conventional techniques. Various automatic synthesizers are commercially available and can be used in accordance with known protocols.

Peptides bound to a polymer support may be prepared by sequential coupling of individual amino acid derivatives by standard techniques *(See, e.g*., Stewart and Young, *Solid Phase Peptide Synthesis* (1984), Pierce Chemical Co., Rockford, IL). Cleavage of the peptide product from the polymer support may be accomplished in a variety of ways dependent upon the type of resin used and the chemical linkage between the peptide and the resin. If, for example, the resin is derived from a polymerized p-alkoxybenzyl alcohol derivative, then cleavage of the peptide-resin linkage may be carried out using a strong acid such as trifluoroacetic acid. Alternatively, if the peptide is synthesized on a polystyrene resin, the cleavage can be accomplished using liquid hydrogen fluoride. If desired, additives such as phenol, anisole and ethanedithiol may be added to the reaction. The crude product thus obtained may be further purified using chromatographic or other methods of chemical purification well known to those of skill in the art.

The peptides may also be prepared by the sequential coupling of amino acid derivatives in solution without the use of polymer resin or other solid supports. The methods useful for solution phase peptide synthesis are well documented in the chemical literature and are known to those skilled in the art.

In the peptides of the invention carbon atoms bonded to four nonidentical substituents are asymmetric. Accordingly, the compounds may exist as diastereoisomers, enantiomers or mixtures thereof. The syntheses described above may employ racemates, enantiomers or diastereomers as starting materials or intermediates. Diastereomeric products resulting from such syntheses may be separated by chromatographic or crystallization methods. Likewise, enantiomeric product mixtures may be separated using the same techniques or by other methods known in the art. Each of the asymmetric carbon atoms, when present in a peptide, may be in one of two configurations (R or S) and both are within the scope of the present invention.

Alternatively, recombinant DNA technology may be employed wherein a nucleotide sequence which encodes a peptide of interest is inserted into an expression vector, transformed or transfected into an appropriate host cell and cultivated under conditions suitable for expression. These procedures are generally known in the art, as described generally in Sambrook et al.²⁹

As the coding sequence for peptides of the length contemplated herein can be synthesized by chemical techniques, for example, the phosphotriester method of Matteucci, et al.³⁰, modification can be made simply by substituting the appropriate base(s) for those encoding the native peptide sequence. The coding sequence can then be provided with appropriate linkers and ligated into expression vectors commonly available in the art, and the vectors used to transform suitable hosts to produce the desired recombinant protein.

The compounds described in this invention may be isolated as or converted to salts of various inorganic and organic acids and bases. Such salts are within the scope of this invention. Examples of such salts include ammonium, metal salts like sodium, potassium, calcium and magnesium; salts with organic bases like dicyclohexylamine, N-methyl-D-glucamine and the like; and salts with amino acids like arginine or lysine. Salts with inorganic and organic acids may be likewise prepared, for example, using hydrochloric, hydrobromic, sulfuric, phosphoric, trifluoroacetic, methanesulfonic, malic, maleic, fumaric and the like. Non-toxic and physiologically compatible salts are particularly useful although other less desirable salts may have use in the processes of isolation and purification.

A number of methods are useful for the preparation of the salts described above and are known to those skilled in the art. For example, reaction of the free acid or free base form of a peptide of the invention with one or more molar equivalents of the desired acid or base in a solvent or solvent mixture in which the salt is insoluble; or in a solvent like water after which the solvent is removed by evaporation, distillation or freeze drying. Alternatively, the free acid or base form of the product may be passed over an ion exchange resin to form the desired salt or one salt form of the product may be converted to another using the same general process.

The peptides employed in the subject invention need not be identical to peptides disclosed in the Example section, below, so long as the subject peptides are able to bind VLA-4 and inhibit intercellular adhesion. Thus, one of skill will recognize that a number of conservative substitutions can be made without substantially affecting the activity of the peptide. Conservative substitutions are those involving replacing an amino acid residue with another which is biologically and/or chemically similar, e.g., one hydrophobic residue for another, or one polar residue for another.

In particular, single amino acid substitutions, deletions, or insertions can be used to determine which residues are relatively insensitive to modification. Substitutions are preferably made with small, relatively neutral moieties such as Ala, Gly, Pro, or similar residues. The effect of single amino acid substitutions may also be probed using D-amino acids. The number and types of residues which are substituted or added depend on the spacing necessary between essential contact points and certain functional attributes which are sought (e.g., hydrophobicity versus hydrophilicity). Increased binding affinity for VLA-4 may also be achieved by such substitutions, compared to the affinity of the parent peptide. In any event, such substitutions should employ amino acid residues or other molecular fragments chosen to avoid, for example, steric and charge interference which might disrupt binding.

The substituting amino acids, however, need not be limited to those naturally occurring in proteins, such as L-α-amino acids, or their D-isomers. The peptides may be substituted with a variety of moieties such as amino acid mimetics well known to those of skill in the art.

In certain embodiments the peptides will include cysteine residues at both termini, which allow the production of cyclic peptides through disulfide linkages. For example, treatment of a such a peptide with an oxidizing agent such as oxygen, iodine or similar agent will produce a cyclic peptide which may be further purified using chromatographic or other methods of chemical purification. These cyclic peptides, which are conformationally restricted, provide probes for determining the spatial requirements at the adhesion signal binding site thereby providing information useful for the design of more potent peptides and non-peptidic molecules. Additionally, cyclic peptides, having the requisite binding affinity described above, are within the scope of this invention.

Construction of cyclic peptides can also be accomplished through thioether linkages. For instance, N-bromoacetyl-derivatized peptides can be reacted with sulfhydryl-containing residues, such as cysteine. Cyclization occurs by reaction of the free sulfhydryl of cysteine in the peptide with the bromoacetyl group to form a thioether linkage (Robey et al.³¹ and U.S. Patent No. 5,066,716³²).

Other methods of constructing cyclic peptides are known to those skilled in the art. These include side chain-side chain, side chain-main chain and main chain-main chain cyclizations. In addition, linkers can be used to join the amino and carboxyl termini of a peptide. The linker is capable of forming covalent bonds to both the amino and carboxyl terminus. Suitable linkers are well known to those of skill in the art and include, but are not limited to, straight or branched-chain carbon linkers, heterocyclic carbon linkers, or peptide linkers. The linkers may be joined to the carboxyl and amino terminal amino acids through their side groups (e.g., through a disulfide linkage to cysteine) or through the alpha carbon amino and carboxyl groups of the terminal amino acids.

In certain embodiments, the peptides will include a cysteine residue only at the C-terminus, which allow for the production of dimeric peptides through disulfide linkages. For example, treatment of such a peptide with an oxidizing agent such as oxygen, iodide or similar agent will produce a dimeric peptide which may be further purified using chromatographic or other methods of chemical purification.

The peptides of the invention can comprise modifications to the N-and/or C-terminal residues. As will be well understood by the artisan, the N-and C-termini may be modified to alter physical or chemical properties of the peptide, such as, for example, to affect binding, stability, bioavailability, ease of linking, and the like.

The N terminal residue can be modified to include acyl groups, in particular acetyl groups. Generally, the modifications will be of the formula R₄-CO- wherein, R₄ is lower alkyl, cycloalkyl, aryl, arylalkyl, or R₅O-. R₅ is lower alkyl, cycloalkyl, aryl or arylalkyl.

Preferred modifications of the C-terminus include modification of the carbonyl carbon of the C-terminal residue to form a carboxy-terminal amide or ester. In general, the amide nitrogen, covalently bound to the carbonyl carbon on the C-terminal residue, will have two substitution groups, each of which can be hydrogen, lower alkyl, aryl, cycloalkyl or alkylaryl group. Where the C-terminal residue is linked to an ester the group will generally have the formula -O-R₆ wherein, R₆ is lower alkyl, cycloalkyl, aryl and arylalkyl.

The N- or C-terminal residue may be linked to a variety of moieties other than amino acids such as polyethylene glycols to increase serum half life and provide other desired properties.

The biological activity of the peptides identified above may be assayed in a variety of systems. For example, a compound can be immobilized on a solid surface and adhesion of cells expressing VLA-4 can be measured. Using such formats, large numbers of specific modifications, (e.g., substitutions, deletions or additions) can be screened. Cells suitable for this assay include any leukocytes known to express VLA-4 such as T cells, B cells, monocytes, and eosinophils, basophils. A number of leukocyte cell lines can also be used, examples include Jurkat, and U937.

The test compounds can also be tested for the ability to competitively inhibit binding between VLA-4 and VCAM-1, or between VLA-4 and a labelled compound known to bind VLA-4, such as peptides of the invention or antibodies to VLA-4. In these assays the VCAM-1 can be immobilized on a solid surface. Alternatively, VCAM-1 expressing cells, such as activated endothelial cells or VCAM-1 transfected fibroblasts can be used. For assays to measure the ability to block adhesion to brain endothelial cells, the assays described in WO 91/05038³³ are particularly preferred.

Many assay formats employ labelled assay components. The labelling systems can be in a variety of forms. The label may be coupled directly or indirectly to the desired component of the assay according to methods well known in the art. A wide variety of labels may be used. The component may be labelled by any one of several methods. The most common method of detection is the use of autoradiography with ³H, ¹²⁵I, ³⁵S, ¹⁴C, or ³²P labelled compounds or the like. Non-radioactive labels include ligands which bind to labelled antibodies, fluorophores, chemiluminescent agents, enzymes, and antibodies which can serve as specific binding pair members for a labelled ligand. The choice of label depends on sensitivity required, ease of conjugation with the compound, stability requirements, and available instrumentation.

Appropriate *in vivo* models for demonstrating efficacy in treating inflammatory responses include EAE in mice, rats or guinea pigs, as well as other inflammatory models dependent upon α4 integrins.

Peptides having the desired biological activity may be modified as necessary to provide desired properties such as improved pharmacological properties (e.g., *in vivo* stability, bio-availability), or the ability to be detected in diagnostic applications. For instance, inclusion of one or more D-amino acids in the peptide typically increases *in vivo* stability, particularly if the D-amino acid residues are substituted at one or both termini of the peptide sequence. Stability can be assayed in a variety of ways such as by measuring the half-life of the proteins during incubation with peptidases or human plasma or serum. A number of such protein stability assays have been described (see, e.g., Verhoef, et al.³⁴).

For diagnostic purposes, a wide variety of labels may be linked to the peptides, which may provide, directly or indirectly, a detectable signal. Thus, the peptides of the subject invention may be modified in a variety of ways for a variety of end purposes while still retaining biological activity. In addition, various reactive sites may be introduced at the terminus for linking to particles, solid substrates, macromolecules, or the like.

Labeled peptides can be used in a variety of *in vivo* or *in vitro* applications. A wide variety of labels may be employed, such as radionuclides (e.g., gamma-emitting radioisotopes such as technetium-99 or indium-111), fluorescers (e.g., fluorescein), enzymes, enzyme substrates, enzyme cofactors, enzyme inhibitors, chemiluminescent compounds, bioluminescent compounds, and the like. Those of ordinary skill in the art will know of other suitable labels for binding to the complexes, or will be able to ascertain such using routine experimentation. The binding of these labels is achieved using standard techniques common to those of ordinary skill in the art.

*In vitro* uses include diagnostic applications such as monitoring inflammatory responses by detecting the presence of leukocytes expressing VLA-4. The peptides can also be used for isolating or labeling such cells. In addition, as mentioned above, the peptides of the invention can be used to assay for potential inhibitors of VLA-4/VCAM-1 interactions.

For *in vivo* diagnostic imaging to identify, e.g., sites of inflammation, radioisotopes are typically used in accordance with well known techniques. The radioisotopes may be bound to the peptide either directly or indirectly using intermediate functional groups. For instance, chelating agents such as diethylenetriaminepentacetic acid (DTPA) and ethylenediaminetetraacetic acid (EDTA) and similar molecules have been used to bind proteins to metallic ion radioisotopes.

The complexes can also be labeled with a paramagnetic isotope for purposes of *in vivo* diagnosis, as in magnetic resonance imaging (MRI) or electron spin resonance (ESR), both of which were well known. In general, any conventional method for visualizing diagnostic imaging can be used. Usually gamma- and positron-emitting radioisotopes are used for camera imaging and paramagnetic isotopes are used for MRI. Thus, the peptides can be used to monitor the course of amelioration of an inflammatory response in an individual. By measuring the increase or decrease in lymphocytes expressing VLA-4 it is possible to determine whether a particular therapeutic regimen aimed at ameliorating the disease is effective.

The pharmaceutical compositions of the present invention can be used to block or inhibit cellular adhesion associated with a number of diseases and disorders. For instance, a number of inflammatory disorders are associated with integrins or leukocytes. Treatable disorders include, e.g., allograft rejection, Alzheimer's disease, atherosclerosis, AIDS demential, diabetes, retinitis, cancer metastases, rheumatoid arthritis, acute leukocyte-mediated lung injury (e.g., adult respiratory distress syndrome), asthma, nephritis, and acute and chronic inflammation, including atopic dermatitis, psoriasis, myocardial ischemia, and inflammatory bowel disease. In preferred embodiments the pharmaceutical compositions are used to treat inflammatory brain disorders, such as multiple sclerosis (MS), viral meningitis and encephalitis.

Pharmaceutical compositions of the invention are suitable for use in a variety of drug delivery systems. Suitable formulations for use in the present invention are found in *Remington's Pharmaceutical Sciences,* Mace Publishing Company, Philadelphia, PA, 17th ed. (1985). For a brief review of methods for drug delivery, see, Langer³⁵.

In order to enhance serum half-life, the peptides may be encapsulated, introduced into the lumen of liposomes, prepared as a colloid, or other conventional techniques may be employed which provide an extended serum half-life of the peptides. A variety of methods are available for preparing liposomes, as described in, e.g., Szoka, et al.³⁶, U.S. Patent Nos. 4,235,871³⁷, 4,501,728³⁸ and 4,837,028³⁹.

The amount administered to the patient will vary depending upon what is being administered, the purpose of the administration, such as prophylaxis or therapy, the state of the patient, the manner of administration, and the like. In therapeutic applications, compositions are administered to a patient already suffering from a disease in an amount sufficient to cure or at least partially arrest the symptoms of the disease and its complications. An amount adequate to accomplish this is defined as "therapeutically effective dose." Amounts effective for this use will depend on the disease condition being treated as well as by the judgment of the attending clinician depending upon factors such as the severity of the inflammation, the age, weight and general condition of the patient, and the like.

The pharmaceutical compositions are intended for parenteral, intranasal, sub-cutaneous, topical, oral or local administration, such as by aerosol or transdermally, for prophylactic and/or therapeutic treatment. Commonly, the pharmaceutical compositions are administered parenterally, e.g., intravenously. Thus, the invention provides compositions for parenteral administration which comprise peptide dissolved or suspended in an acceptable carrier, preferably an aqueous carrier, e.g., water, buffered water, saline, PBS and the like. The compositions may contain pharmaceutically acceptable auxiliary substances as required to approximate physiological conditions, such as pH adjusting and buffering agents, tonicity adjusting agents, wetting agents, detergents (e.g., nonionic surfactants such as Tween, Pluronics) and the like, for example, sodium acetate, sodium lactate, sodium chloride, potassium chloride, calcium chloride, sorbitan monolaurate, and triethanolamine oleate. In addition the compositions may comprise antioxidants such as ascorbic acid; low molecular weight (less than about ten residues) peptides such as polyarginine, proteins, such as serum albumin, gelatin, or immunoglobulins; hydrophilic polymers such as polyvinylpyrrolidinone; amino acids such as glycine, glutamic acid, aspartic acid, or arginine; monosaccharides, disaccharides, and other carbohydrates including cellulose or its derivatives, glucose, mannose, or dextrins; chelating agents such as EDTA; and sugar alcohols such as mannitol or sorbitol.

These compositions may be sterilized by conventional sterilisation techniques, or may be sterile filtered. The resulting aqueous solutions may be packaged for use as is, or lyophilized, the lyophilized preparation being combined with a sterile aqueous carrier prior to administration. The pH of the peptide preparations typically will be between 3 and 11, more preferably from 5 to 9 and most preferably from 7 and 8. It will be understood that use of certain of the foregoing excipients, carriers, or stabilizers will result in the formation of peptide salts.

The concentration of peptides in the pharmaceutical formulations can vary widely, i.e., from less than about 0.01%, usually at or at least about 5% to as much as 50 to 75% by weight and will be selected primarily by fluid volumes, viscosities, etc., in accordance with the particular mode of administration selected.

The therapeutic dosage of the peptides of the present invention will vary according to, for example, the particular use for which the treatment is made, the manner of administration of the peptides, the health and condition of the patient, and the judgment of the prescribing physician. For example, for intravenous administration, the dose will typically be in the range of about 20µg to about 500µg per kilogram body weight, preferably about 100µg to about 300µg per kilogram body weight. Suitable dosage ranges for intranasal administration are generally about 0.1pg to 1 mg per kilogram body weight. Effective doses can be extrapolated from dose-response curves derived from *in vitro* or animal model test systems.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, preferably 25-75%.

For aerosol administration, the peptides are preferably supplied in finely divided form along with a conventional non-toxic surfactant and a suitable propellant. Typical percentages of peptides are 0.01%-20% by weight, preferably 1%-10%; and of surfactant from 0.1%-20% by weight, preferably 0.25%-5%.

Two or more peptides of the invention may be combined to form a peptide "cocktail" under certain circumstances for increased efficacy. The peptides of the invention may also be used in conjunction with other pharmaceutically active agents.

The following examples are offered to illustrate this invention and are not to be construed in any way as limiting the scope of this invention. Unless otherwise stated, all temperatures are in degrees Celsius. Also, in these examples, unless otherwise defined below, the abbreviations employed have their generally accepted meaning:
- Å: = Angstroms
- BSA: = bovine serum albumin
- cm: = centimeter
- DCC: = N,N-dicyclohexylcarbodiimide
- DMF: = dimethylformamide
- E-64: = trans-epoxysuccinyl-L-leucylamido-(4- guanidino)butane
- FACS: = fluorescent activated cell sorter
- FMOC: = fluorenylmethoxycarbonyl
- HBTU: = O-benzotriazol-1-yl-N,N,N',N'- tetramethyluronium hexaflurophosphate
- HOBT: = 1-hydroxybenzotriazole
- HPLC: = high performance liquid chromatography
- I.D.: = internal diamter
- MBHA: = methyl benzhydrylamine
- mg: = milligram
- mL: = milliliter
- mmole: = millimole
- mM: = millimolar
- N: = normal
- PAM: = phenylacetamidomethyl
- PMSF: = phenylmethylsulfonyl fluoride (α-toluenesulfonyl fluoride)
- TBTU: = 2-(1H-benzotriazol-1-yl)-1,1,3,3- tetramethyluronium
- t-BOC: = t-butyloxycarbonyl
- TFA: = trifluoroacetic acid
- µg: = micrograms
- µL: = microliters
- µm: = micron

### Example 1

### Peptide synthesis using fluorenylmethoxycarbonyl (FMOC) protected amino acids

Peptides were synthesized on an automated Symphony Multiplex Peptide Synthesizer from Protein Technologies using instrument resident software. The syntheses were carried out on a 0.1 mmole scale utilizing FMOC protected amino acids. 4-Hydroxymethylphenoxymethyl polystyrene resin was used to obtain C-terminal carboxylic acid peptides and 4-(2',4'-dimethoxyphenyl-FMOC-aminomethyl)phenoxymethyl-polystyrene resin was used to produce C-terminal peptide amides. Amino acid side chains were protected as follows: Arg(2,2,5,7,8-pentamethylchroman-6-sulfonyl), Asp(t-butoxy), Glu(t-butoxy), Ser(t-butyl), Thr(t-butyl), Tyr(t-butyl), Asn(trityl), Gln(trityl), His(trityl), Lys(t-butoxycarbonyl), Cys (S-trityl).

FMOC amino acids were dissolved in DMF to 200 mM concentrations. Each FMOC amino acid was activated, *in situ,* to its corresponding active ester using 200 mM HBTU/0.4 mM N-methyl-morpholine in DMF. Following removal of the FMOC protecting group from the previous amino acid addition using 20% piperidine in DMF, a five fold excess of the FMOC amino acid was added followed by an equimolar amount of activator. The amino acid active ester was formed *in situ* and allowed to react for twenty minutes. The peptide resin was then washed in DMF and dichloromethane and any uncoupled amine was capped using 10% acetic anhydride in dichloromethane. At this point the synthesis cycle was repeated beginning with FMOC deprotection.

After the synthesis cycles were complete, the final FMOC group was removed with 20% piperidine in DMF and at this point the N-terminal was N-acetylated if so desired. Each peptide resin was then cleaved from the resin support and stripped of any remaining side chain protecting groups by treatment for 2 hours with 50% TFA in dichloromethane. Crude peptides were precipitated by the addition of cold diethyl ether. The precipitate was centrifuged, the solvent removed, and the precipitate washed with cold diethyl ether and centrifuged two more times. The crude peptide precipitate was then dissolved in aqueous 2N acetic acid and lyophilized. The peptides produced using this automated FMOC protocol were purified as described in Example 2 for those peptides produced using t-BOC chemistry.

### Example 2

### Peptide synthesis using t-BOC protection

Peptides were synthesized on an Applied Biosystems Model 430A Peptide Synthesizer using modified double couple capping cycles for each amino acid addition. The syntheses were carried out on a 0.5 mmole scale. Peptides which are C-terminal carboxylic acids were synthesized on PAM polystyrene resins which give the desired peptide acid following HF treatment. C-terminal peptide amides were synthesized on MBHA polystyrene resin. Amino acid side chain protection was as follows: Arg(p-toluenesulfonyl), Asp(benzyl), Glu(benzyl), Cys(4-methylbenzyl), His(t-butyloxymetyl), Lys(4-chlorobenzyloxycarbonyl), Ser(benzyloxy), Thr(benzyloxy), Tyr(4-bromobenzyloxycarbonyl). All other amino acids were used with no further side chain protection.

Amino acids were coupled following preactivation to the symmetric anhydride using 0.5 equivalent of DCC in dichloromethane. Glutamine and asparagine were preactivated to their respective HOBT esters using 1 equivalent of DCC and 1 equivalent of HOBT in dimethylformamide. Capping of any uncoupled amine with 10% acetic anhydride in dichloromethane followed the recoupling step for each amino acid addition. The capping protocol was used to minimize synthesis of deletion peptides which are often difficult to separate from the target peptide. The BOC protecting group on each amino acid α-amine was removed with 50% trifluoroacetic acid in dichloromethane. Following removal of the BOC group on the final amino acid and N-terminal acetylation if desired, the peptides were cleaved from the resin support with anhydrous hydrogen fluoride which concurrently removes any remaining side chain protecting groups. The HF cleavage and deprotection was accomplished in 1 hour at 0°C in the presence of anisole as a scavenger and with the addition of methylethylsulfide for peptides containing methionine or cysteine. Following HF treatment, the peptide was extracted from the resin in 2N acetic acid and lyophilized.

The crude peptide was then purified using preparative reverse phase HPLC on Vydac C4 or C18, 330Å, 10 µm columns that are 2.2 cm I.D. x 25 cm in length. The system of choice was 0.1 % TFA/H₂O (A buffer) and 0.1% TFA/CH₃CN (B buffer) as the mobile phase. Typically the peptide was loaded onto the column in 2% B buffer at 8-10 mL/minute and eluted using a linear gradient of 2% B buffer to 60% B buffer in 174 minutes.

The purified peptide was characterized by amino acid analysis, mass spectrometry, and analytical reverse phase HPLC and, if required, sequence analysis.

### Example 3

### Disulfide bonded cyclic and dimeric peptides

Peptides synthesized in Examples 1 and 2 which contain cysteine residues were cyclized or dimerized via the formation of either an intramolecular (cyclic form) or intermolecular (dimeric form) disulfide bond. The crude peptide, following cleavage from the resin and lyophilization from 2N acetic acid, was made to a concentration of 1 mg/mL in glacial acetic acid. A ten-fold molar excess of iodine, dissolved in the minimum amount of glacial acetic acid, was added dropwise to the peptide solution over a period of ca. 60 minutes. Upon completion of the addition, the reaction was continued for 4 hours at room temperature. The reaction solution was then treated with zinc dust to consume unreacted iodine, filtered and concentrated. The residual product was lyophilized from 2N acetic acid. The crude product was then purified as described in Example 2.

The peptides were also cyclized or dimerized by air oxidation to form disulfide bonds. In this case, the peptide was dissolved at ≈0.5mg/mL in NH₄OAc pH 8.5 and stirred gently in a flask open to the air. The reaction progress was monitored by HPLC and Ellman analysis to detect free sulfhydryl groups.

Dimerization occurs when the peptide contains a single cysteine residue whereas cyclization, as per above, can occur when the peptide contains two or more cysteine residues.

### Example 4

### Thioether cyclization

Peptides having a C-terminal Cys residue and an N-terminal Br-Acetyl- can be cyclized via formation of an intramolecular thioether *(see,* Robey et al.³¹).

To form the thioether, the pH of the peptide in 200-500 mL aqueous acetic acid was adjusted to 8.5 by slowly adding NH₄OH (ammonium hydroxide). When the pH was stabilized, the solution was stirred for at least 4 hours at room temperature. When thioether formation was complete, the solution was loaded directly onto a preparative HPLC column for desalting or lyopholized.

### Example 5

### Adhesion of Jurkat cells to Peptide-BSA conjugates

This example demonstrates that a peptide derived from the antibody 21/6 CDR3 region interacts specifically with VLA-4 integrin.

The peptide used in this example was synthesized based on the sequence of the anti-α₄ integrin antibody 21/6 (*CGG*EGYYGNYGVYA, SEQ ID No. 1). In this example the peptide was conjugated to a large carrier protein via the linker region that was synthesized as part of the peptide itself (in italics). The conjugated peptide was then coated onto plastic, and the ability of the conjugate to support cell adhesion was tested. This method is based on a report by Wayner and Kovach⁴⁰.

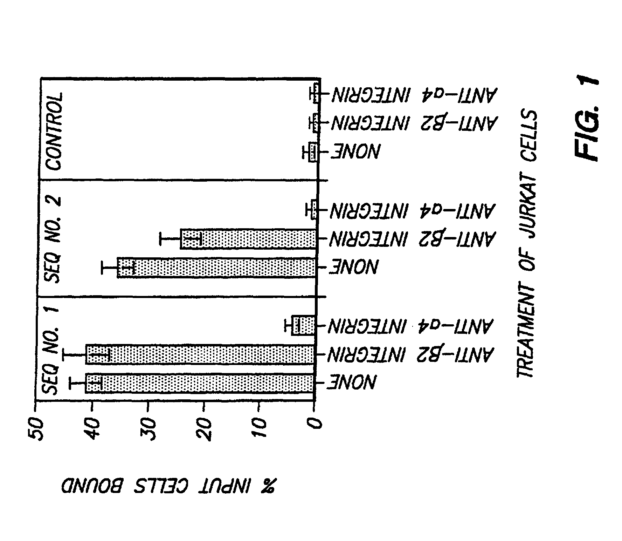

As a positive control, the fibronectin peptide described by Wayner was synthesized, conjugated to carrier protein, and tested for cell adhesion in parallel with SEQ ID NO. 1 (*CGG*EILDVPST, SEQ ID No. 2). Both peptide conjugates were exposed to the plastic at a concentration of 3 µg/mL. As shown in Figure 1, Jurkat cells bound equally well to the two peptides, but did not bind control protein. Adhesion was not affected by an antibody against β₂ integrin (IOT18), a distinct cell adhesion molecule that is also expressed on the surface of Jurkat cells. Thus, like peptide SEQ ID NO. 2, peptide SEQ ID NO. 1 interacts with Jurkat in an α₄ integrin-specific fashion.

### Example 6

### Jurkat Interaction with Soluble VCAM-1

This example demonstrates that peptides derived from the antibody 21/6 CDR3 region can be used as competitive inhibitors of VLA-4 integrin interaction with VCAM-1.

VCAM-1 was expressed as a soluble fusion protein. The construct expressed the seven extracellular domains of VCAM-1 on the N-terminus, and the human IgG₁ heavy chain constant region on the C-terminus. Interaction of the soluble construct with Jurkat cells was monitored by standard FACS analysis, using a fluorescently labeled antibody directed against the construct's human IgG tail as a marker.

The activity of VLA-4 can be regulated. 15/7 is a monoclonal antibody that recognizes an activated conformation of VLA-4 and locks the molecule in the active state, thereby enhancing VLA-4-mediated cell adhesion. 15/7 stabilizes the interaction of Jurkat cells with the soluble VCAM-1 construct.

When mixed with Jurkat cells in the presence of Mn⁺² and 15/7, the soluble VCAM-1 construct interacted with the cell surface in an VLA-4-dependent fashion; the interaction was inhibited completely by anti-α4 integrin (21/6), but not by the IOT18 antibody against β₂ integrin (data not presented).

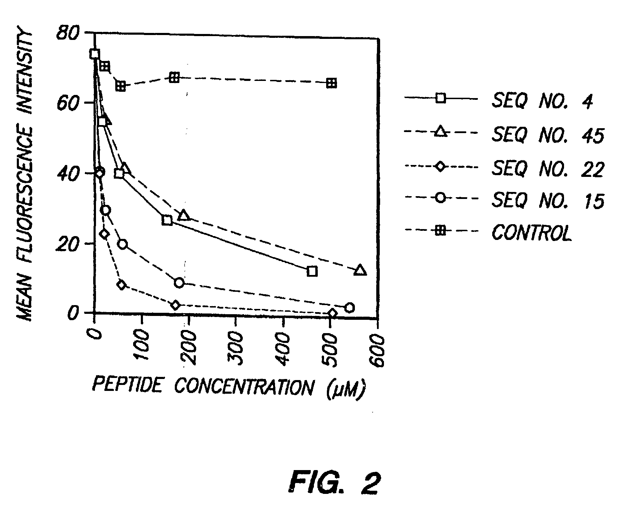

Figure 2 shows that peptide SEQ ID No. 4, derived from the CDR3 region of the anti-α4 integrin antibody 21/6 (EGYYGNYGVYA), inhibited interaction of sVCAM-1-IgG with Jurkat cells in a dose-dependent fashion. Truncation of the first two amino acids from the peptide's N-terminus had no significant effect on activity (SEQ ID No. 27), whereas truncation of the last alanine greatly increased inhibitory activity (SEQ ID No. 17).

### Example 7

### Peptides from the sequence of antibody 21/6

This example summarizes data from several studies that examined the effect of specific peptide modifications on the activity of peptides derived from the antibody 21/6.

Peptides of different length, and with various amino acid substitutions were synthesized based on the sequence of the CDR3 region of the antibody 21/6 (EGYYGNYGVYA) (SEQ ID No. 4). The peptides were then tested for their ability to inhibit Jurkat interaction with sVCAM-1-IgG in the FACS assay described in Example 6. IC₅₀ values were determined for each peptide using this assay.

The sequence of peptides tested is provided in Table 1. N-acetylation is indicated by "[Ac]". This table also includes the expected and observed mass of each peptide used in the assays.

In one series of experiments, various amino acids were substituted with alanine (A) to determine the importance of the native amino sequence on the peptide's activity. These experiments demonstrated that both N-terminal tyrosines were crucial for activity, but that the other two tyrosines were not. In fact, substitution of the C-terminal tyrosine greatly enhanced the peptide's activity. In another series of experiments, amino acids were deleted from the N and C-terminus to determine the minimal length of the peptide required for activity. These results demonstrated that removal of the C-terminal alanine greatly increased the peptide's activity, but that activity was diminished by further deletions. These experiments define an eight amino acid domain (YYGNYGVY) that is important for activity, and two crucial amino acids within this domain (the N-terminal YY). A short cyclic peptide with homology to this peptide (CYF*Q*NC; pressinoic acid, SEQ ID No. 29) was also tested.

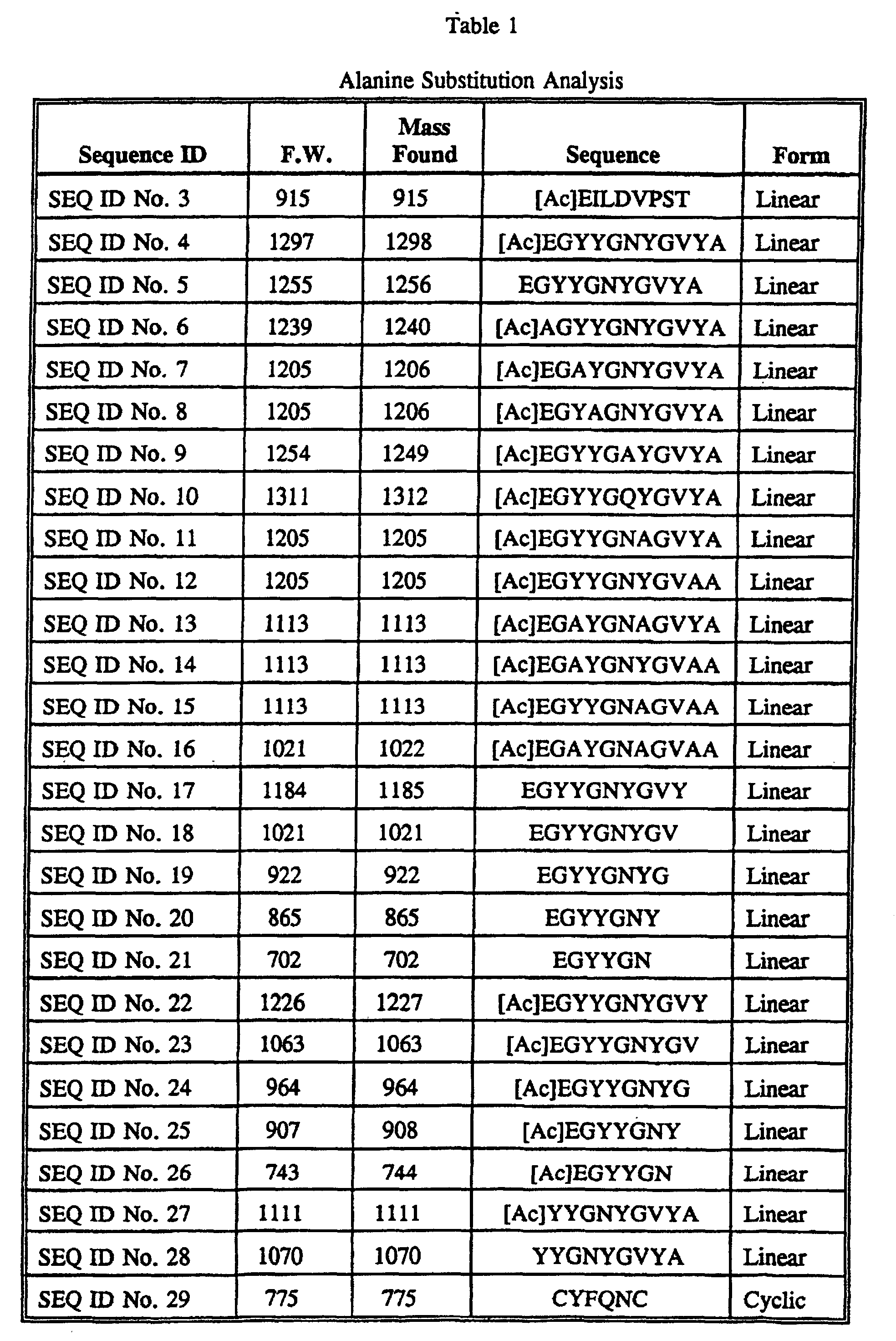

### Example 8

### Peptides derived from VCAM-1 Inhibit Jurkat Interaction with Soluble VCAM-1

This example demonstrates that specific peptides derived from the first domain of VCAM-1 can be used as competitive inhibitors of VLA-4 interaction with VCAM-1.

Peptides derived from the FGN region of VCAM-1 described above were tested as competitive inhibitors of Jurkat cell interaction with VCAM-1 (as described in example 6). The results are shown in Table 2, below. As in Table 1, N-acetylation is indicated by [Ac]. C-terminal amidation is indicated by [NH₂]. One peptide (SEQ ID No. 31) showed particularly good inhibition. Peptides from other regions of VCAM-1 were also tested, two of which were found to be inhibitory (SEQ ID No. 34 and SEQ ID No. 40). SEQ ID No. 34 contains the QID tripeptide which has previously been identified⁴¹ as important for the interaction of VCAM-1 with VLA-4 integrin. A cyclic peptide derived from this sequence was more active than the linear peptide (SEQ ID No. 35). SEQ ID No. 40 is from a region of VCAM-1 adjacent to the FGN motif described above (the C-terminus of this peptide carries the F from FGN). This peptide has not been previously described and is quite active in a linear form. Two preferred peptides are cyclic peptides, SEQ ID Nos. 53 and 59.

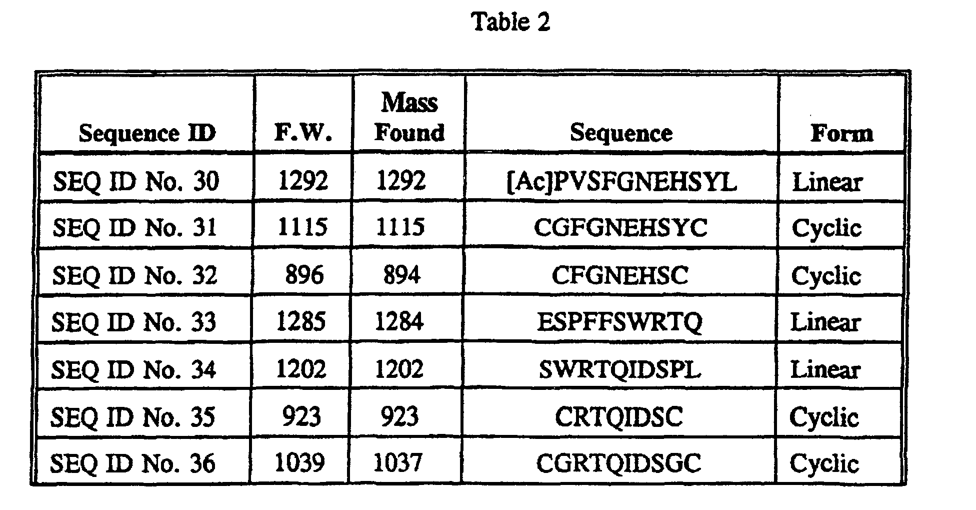

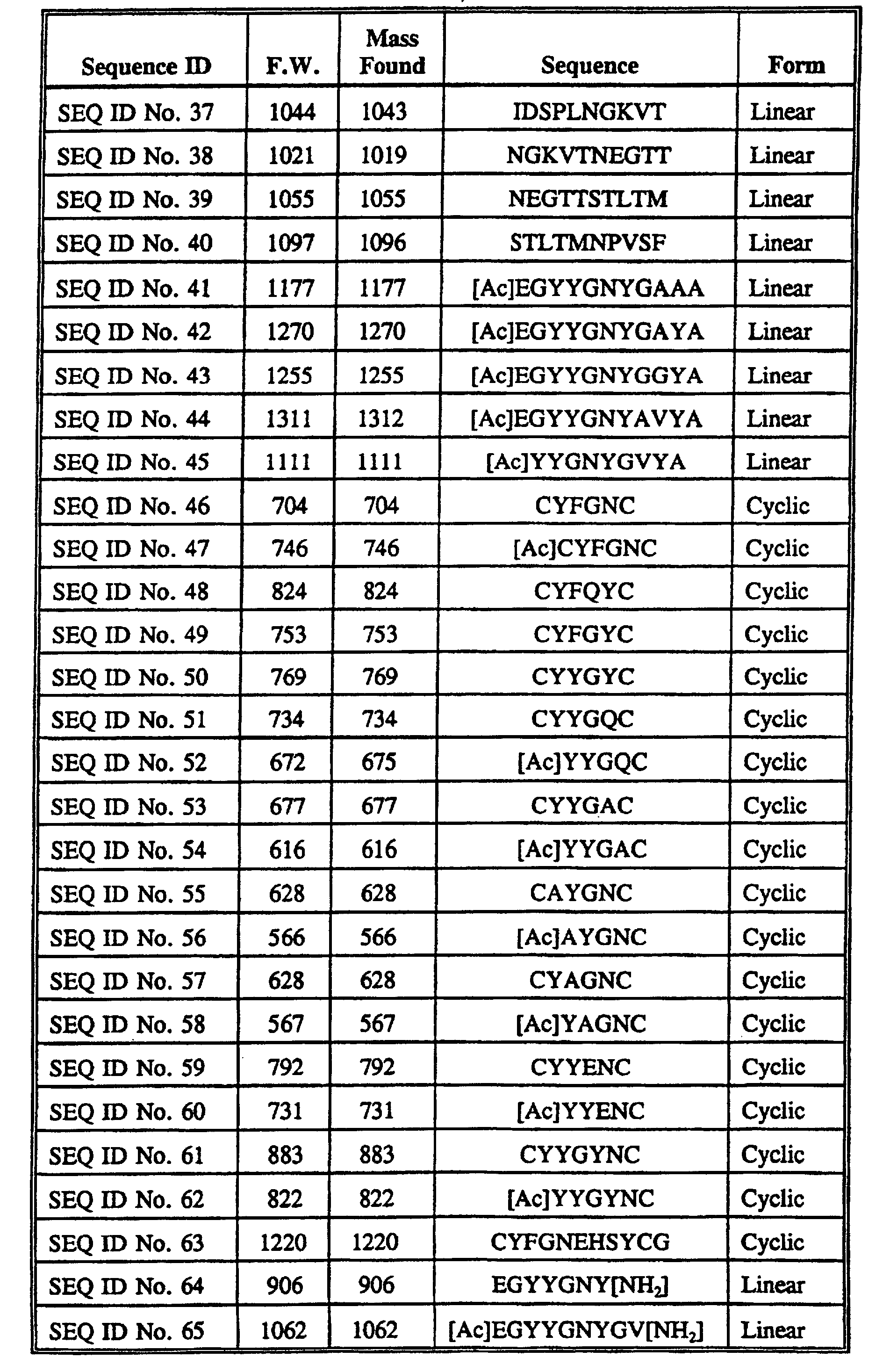

### Example 9

### Peptide Derivatives

Other peptides, derived from those of Tables 1 and 2 above, were prepared and are set forth in Table 3 below:

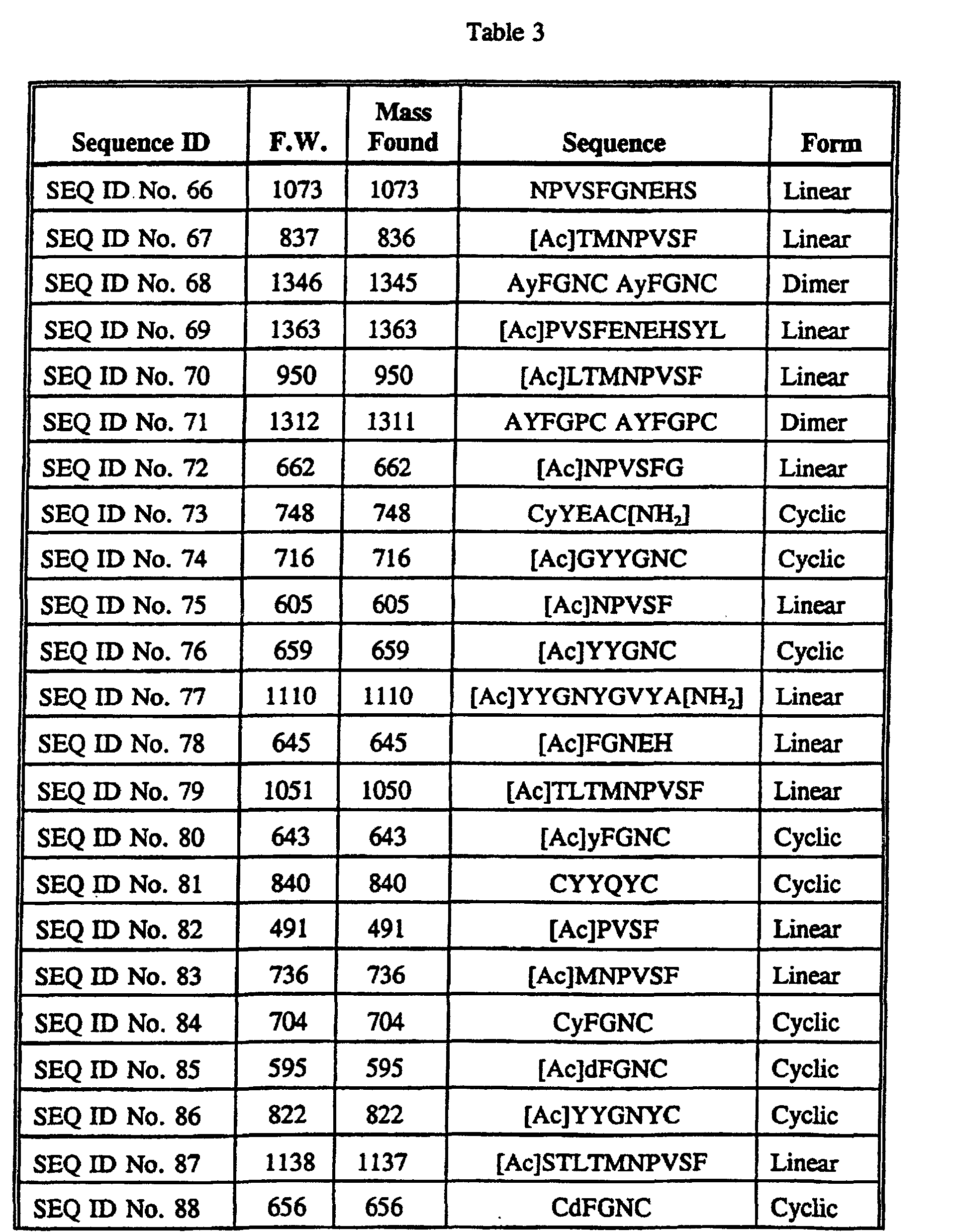

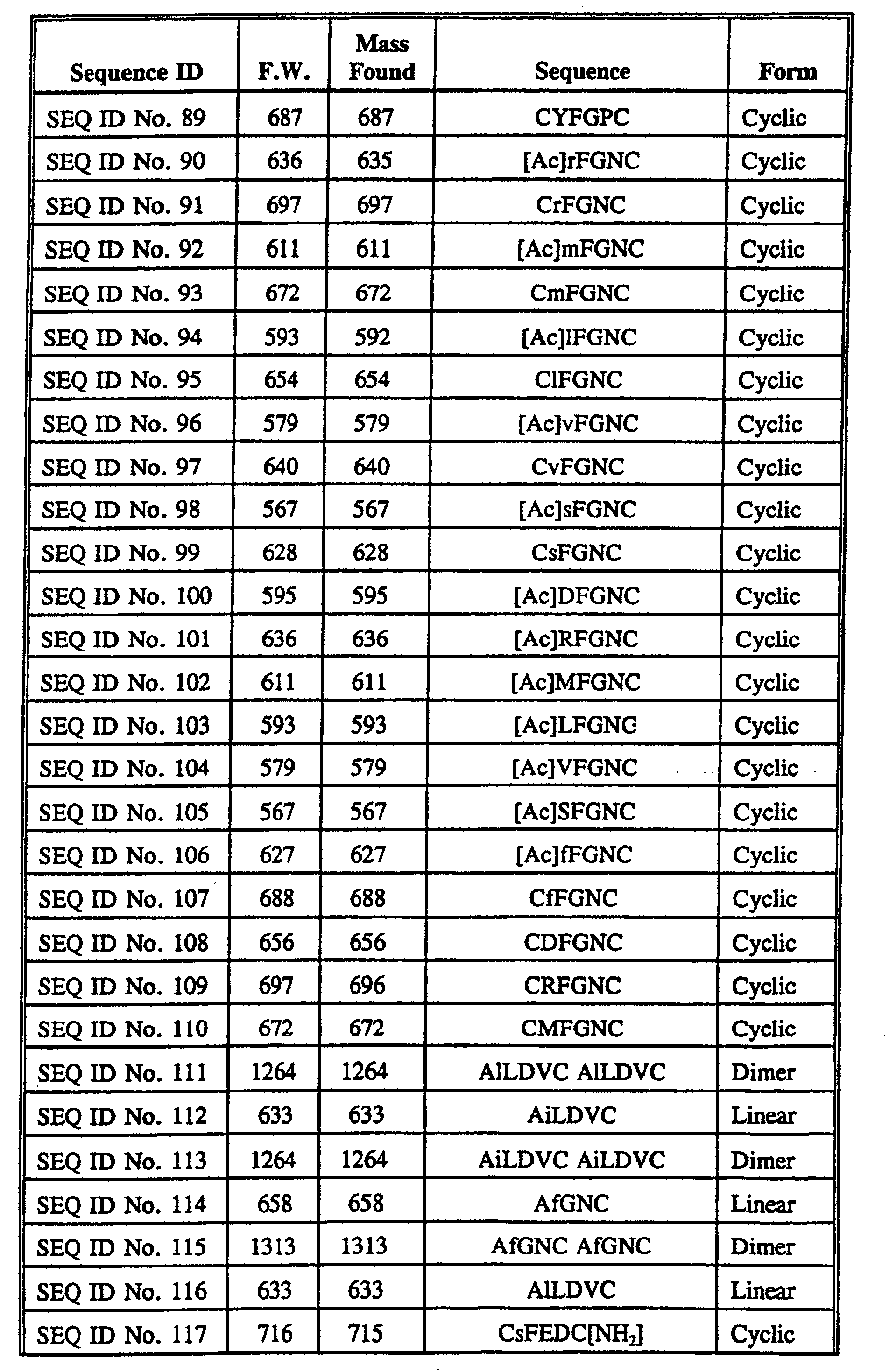

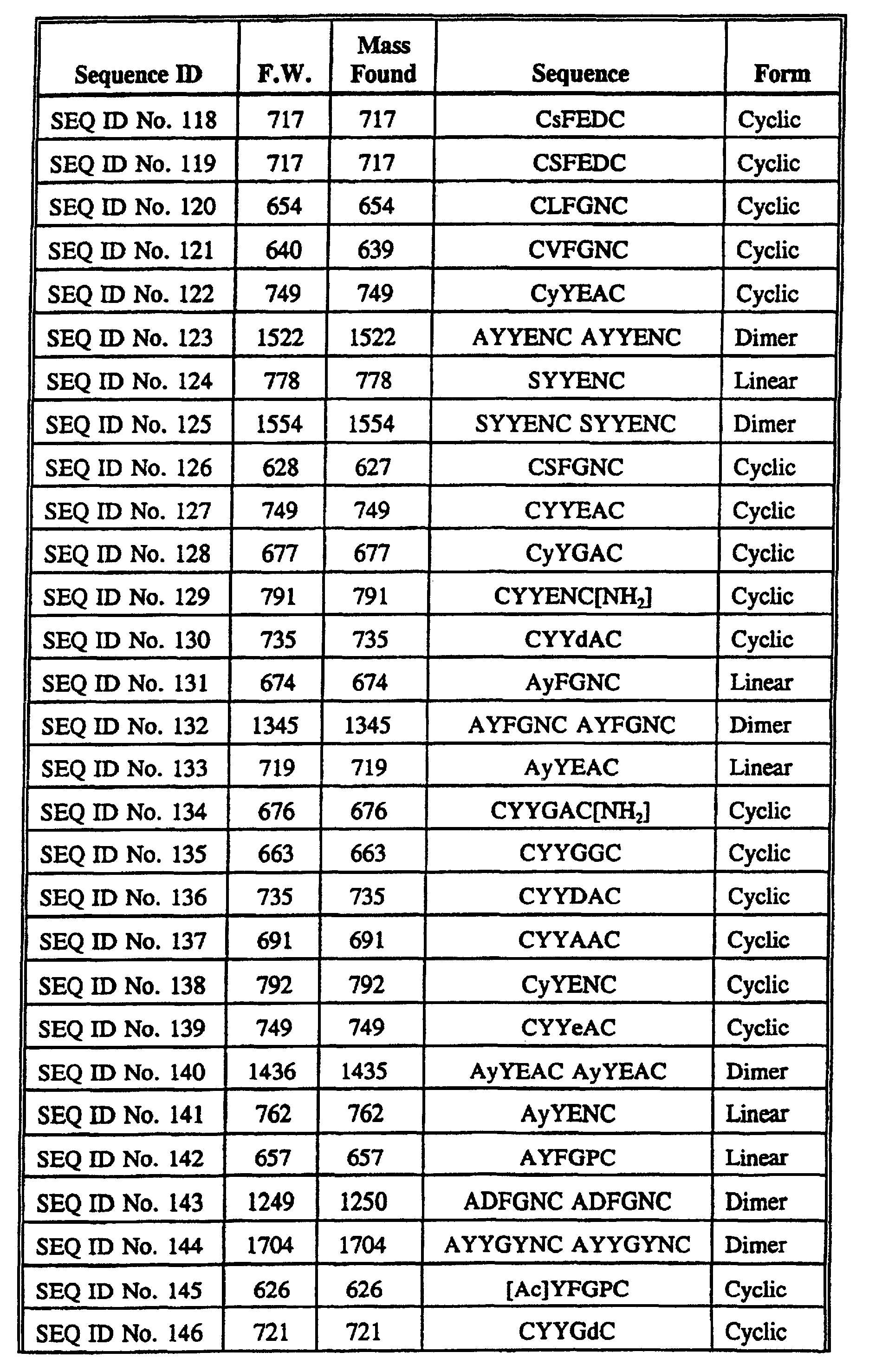

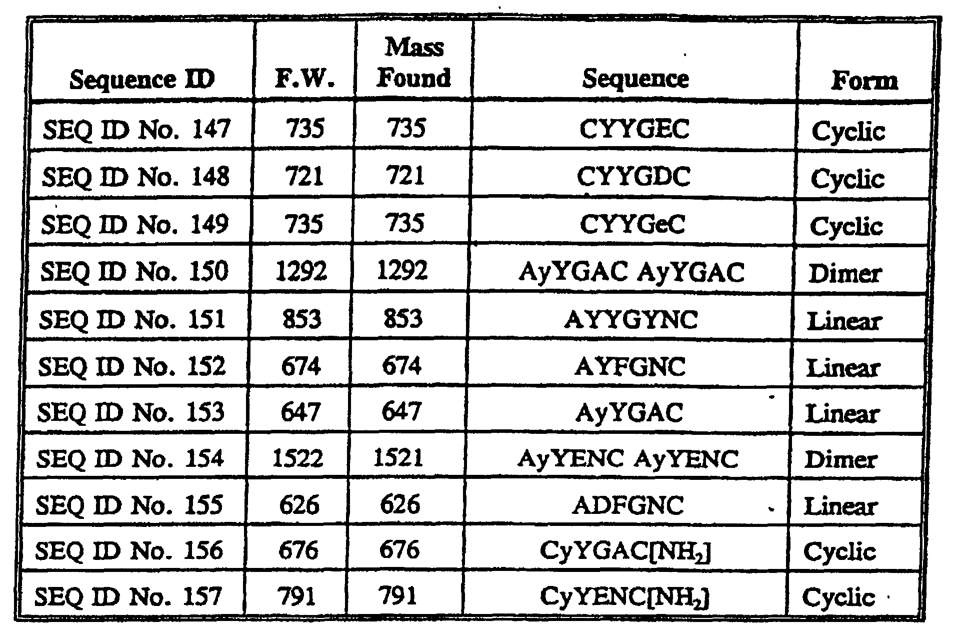

In the tables above, peptides recited as "cyclic" having a [Ac] group at the N-terininus are cyclic thioethers of the general formula: 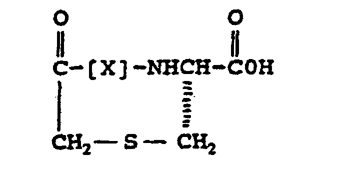 where [X] represents the residues between the N-terminal [Ac] and the C-terminal cysteine residues.

Of the peptides set forth in Tables 1, 2 and 3 above, peptides defined by SEQ ID Nos. 4, 9, 12, 15, 17, 22, 31, 32, 35, 40, 41, 42, 43, 45, 52, 54, 60, 62, 63, 71, 94, 96, 123, 124, 125, 133, 140, 142, 144, 150, 154 each bound to VLA-1 and had an IC₅₀ ( per Example 6 above) of 50 µM or less.

### Example 10 (COMPARATIVE EXAMPLE)

### Cell Free Assay to Measure the Interaction of α₄β₁ Integrin With VCAM-1

In this assay, Jurkat cells are lysed with a detergent to solubilize α₄β₁ integrin [15 x 10⁶ Jurkat cell/mL, 1% n-octyl β-D-glucopyranoside (Sigma #0-8001) with 150 mM NaCl, 1 mM CaCl₂, 1 mM MgCl₂, 1 mM MnCl₂, pH 7.4, and the protease inhibitors PMSF, E-64, and leupeptin]. An antibody against β₁ integrin, TS2/16 (ATCC #HB-243, from the American Type Tissue Collection, Rockville, Maryland, USA) is added to the lysate. TS 2/16 is an activating antibody against β₁ integrin that promotes β₁ integrin-dependent interactions^{42,43}, thereby increasing the activity of solubilized α₄β₁ integrin in the assay. The ascites was diluted 1:1000-1:3000 in the assay.

200 µL of the lysate (with TS2/16) is added to microtiter wells (on a 96 well plate) and 20 µL of 10X test compound is added to the lysate for 30 minutes at 4°C. 50 µL of the treated lysate is added to triplacate wells of a 96 well plate coated with sVCAM-1-IgG (wells of a 96 well plate are coated with sVCAM-1-IgG, 10 µg/mL, 50 µL/well, overnight at 4°C and the wells then blocked with 1% BSA), and then adhesive incubation is allowed to occur for 30 minutes at room temperature. The plate is then washed, blocked with 1% BSA, and the exposed to alkaline phosphatase-conjugated goat anti-mouse Ig (BioRad #170-6520), which recognizes TS2/16 associated with α₄β₁ integrin that has bound sVCAM-1-IgG on the assay well. After 30 minutes at room temperature, the wells are washed and exposed to a substrate for alkaline phosphatase (2-amino-2-methyl-1-propanol) to quantify the amount of α₄β₁ integrin that has bound the sVCAM-1-IgG.

The results of the IC₅₀ values for several peptides described herein are comparable with the IC₅₀ values obtained via the assay of Example 6 above.

## Claims

1. A peptide having a binding affinity to VLA-4 as expressed by an IC₅₀ of about 50 µM or less which peptide is selected from the group consisting of peptides defined by the formula :
[R¹-Y/F-G/E-R²]ₙ
that,comprises an amino acid sequence YYGN ;
and peptides of the formula :
R-PVSF-R'
where R and R' are independently peptide sequences of from 0 to 7 amino acid residues with the proviso that the sum of amino acid residues in R+R' is no more than 9,
R¹ is a peptide sequence of from 0 to 6 amino acid residues and R² is a peptide sequence of from 1 to 7 amino acid residues with the proviso that the sum of amino acid residues in R¹+R² is from 2 to 11, and
n is an integer equal to either 1 or 2.

2. The peptide of claim 1, wherein one or more of the amino acid residues are D-amino acid residues.

3. The peptide of claim 1, wherein the N-terminus of said amino acid residue is modified by linkage of the amine to a group R₄-CO- wherein, R₄ is hydrogen, alkyl of 1-10 carbon atoms, cycloalkyl, aryl, arylalkyl.

4. The peptide of claim 1, wherein the N-terminus of said amino acid residue is modified by linkage of the amine to a group R₅O- where R₅ is alkyl of 1-10 carbon atoms, cycloalkyl, aryl, arylalkyl.

5. The peptide of claim 1, wherein the C-terminus of said peptide is modified by linkage of the carbonyl [C(O)] to a group having the formula: 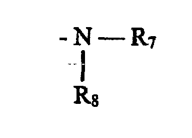 wherein R₇ and R₈ are the same or different and are selected from the group consisting of hydrogen, alkyl of 1-10 carbon atoms, cycloalkyl, aryl or arylalkyl

6. The peptide of claim 1, wherein the C-terminal residue of said peptide is linked to a group having the formula -O-R₆ wherein R₆ is selected from the group consisting of alkyl of 1-10 carbon atoms, cycloalkyl, aryl or arylalkyl.

7. The peptide of claim 3, wherein the C-terminal residue of said peptide is linked to a group having the formula -O-R₆ wherein R₆ is selected from the group consisting of alkyl of 1-10 carbon atoms, cycloalkyl, aryl or arylalkyl.

8. The peptide of claim 4, wherein the C-terminal residue of said peptide is linked to a group having the formula -O-R₆ wherein R₆ is selected from the group consisting of alkyl of 1-10 carbon atoms, cycloalkyl, aryl or arylalkyl.

9. The peptide of claim 1, wherein the peptide consist of between about 6 and about 10 amino acid residues.

10. The peptide of claim 1, wherein the peptide consist of between about 8 and about 10 amino acid residues.

11. The peptide of claim 1, wherein the peptide comprises C at the C-terminus.

12. The peptide of claim 11, wherein the peptide is dimeric.

13. The peptide of claim 1, wherein the peptide is selected from the group consisting of: SEQ ID Nos. 4, 9, 12, 15, 17, 22, 31, 32, 40, 41, 42, 43, 45, 52, 54, 60, 62, 63, 71, 94, 96, 123, 124, 125, 133, 140, 142, 144, 150 and 154.

14. The peptide of claim 1, which includes the sequence YYGNYGVY.

15. The peptide of claim 1, wherein the peptide is selected from the group consisting of SEQ ID Nos. 4, SEQ ID Nos. 17, SEQ ID Nos. 22 and SEQ ID Nos. 45.

16. A pharmaceutical composition comprising a pharmaceutically acceptable carrier and a therapeutically effective amount of a peptide of claim 1.

17. The pharmaceutical composition of claim 16, wherein the peptide is selected from the group consisting of: SEQ ID Nos. 4, 9, 12, 15, 17, 22, 31, 32, 40, 41, 42, 43, 45, 52, 54, 60, 62, 63, 71, 94, 96, 123, 124, 125, 133, 140, 142, 144, 150 and 154.

18. The use of a composition comprising a pharmaceutically acceptable carrier and a therapeutically effective amount of a peptide of claim 1 for the manufacture of a medicament.

19. The use of a composition comprising a pharmaceutically acceptable carrier and a therapeutically effective amount of a peptide of claim 1 for the manufacture of a medicament for treating an inflammatory disease in a patient.

20. The use of claim 19, wherein the inflammatory disease is an inflammatory brain disorder.

21. The use of claim 19, wherein the inflammatory brain disorder is multiple sclerosis.

22. The use of a composition comprising a peptide of claim 1 for the manufacture of a medicament for inhibiting the adhesion of leukocytes mediated by VLA-4.

## Patentansprüche

1. Peptid mit einer Bindungsaffinität für VLA-4, ausgedrückt durch einen IC₅₀ von ungefähr 50 µM oder weniger, das ausgewählt ist aus der Gruppe bestehend aus Peptiden, die durch die Formel:
[R¹-Y/F-G/E-R²]ₙ,
definiert sind, die eine Aminosäuresequenz YYGN umfasst;
und Peptiden mit der Formel:
R-PVSF-R',
wobei R und R' unabhängig Peptidsequenzen mit von 0 bis 7 Aminosäureresten sind, unter der Voraussetzung, dass die Summe der Aminosäurereste in R+R' nicht größer als 9 ist,
R¹ eine Peptidsequenz mit von 0 bis 6 Aminosäureresten und R² eine Peptidsequenz mit von 1 bis 7 Aminosäureresten ist, unter der Voraussetzung, dass die Summe der Aminosäurereste in R¹+R² von 2 bis 11 beträgt, und n eine ganze Zahl gleich entweder 1 oder 2 ist.

2. Peptid nach Anspruch 1, wobei ein oder mehrere der Aminosäurereste D-Aminosäurereste sind.

3. Peptid nach Anspruch 1, wobei der N-Terminus des Aminosäurerestes durch Verknüpfung des Amins mit einer Gruppe R₄-CO- modifiziert ist, worin R₄ Wasserstoff, Alkyl mit 1-10 Kohlenstoffatomen, Cycloalkyl, Aryl, Arylalkyl ist.

4. Peptid nach Anspruch 1, wobei der N-Terminus des Aminosäurerestes durch Verknüpfung des Amins mit einer Gruppe R₅O- modifiziert ist, wobei R₅ Alkyl mit 1-10 Kohlenstoffatomen, Cycloalkyl, Aryl, Arylalkyl ist.

5. Peptid nach Anspruch 1, wobei der C-Terminus des Peptids durch Verknüpfung des Carbonyls [C(O)] durch Verknüpfung mit einer Gruppe der Formel: 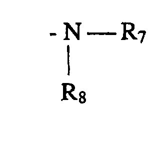 modifiziert ist, wobei R₇ und R₈ gleich oder unterschiedlich sind und ausgewählt sind aus der Gruppe, die aus Wasserstoff, Alkyl mit 1-10 Kohlenstoffatomen, Cycloalkyl, Aryl oder Arylalkyl besteht.

6. Peptid nach Anspruch 1, wobei der C-terminale Rest des Peptids mit einer Gruppe mit der Formel -O-R₆ verknüpft ist, wobei R₆ ausgewählt ist aus der Gruppe, die aus Alkyl mit 1-10 Kohlenstoffatomen, Cycloalkyl, Aryl oder Arylalkyl besteht.

7. Peptid nach Anspruch 3, wobei der C-terminale Rest des Peptids mit einer Gruppe mit der Formel -O-R₆ verknüpft ist, wobei R₆ ausgewählt ist aus der Gruppe, die aus Alkyl mit 1-10 Kohlenstoffatomen, Cycloalkyl, Aryl oder Arylalkyl besteht.

8. Peptid nach Anspruch 4, wobei der C-terminale Rest des Peptids mit einer Gruppe der Formel -O-R₆ verknüpft ist, wobei R₆ ausgewählt ist aus der Gruppe, die aus Alkyl mit 1-10 Kohlenstoffatomen, Cycloalkyl, Aryl oder Arylalkyl besteht.

9. Peptid nach Anspruch 1, wobei das Peptid aus zwischen ungefähr 6 und ungefähr 10 Aminosäureresten besteht.

10. Peptid nach Anspruch 1, wobei das Peptid aus zwischen ungefähr 8 und ungefähr 10 Aminosäureresten besteht.

11. Peptid nach Anspruch 1, wobei das Peptid C am C-Terminus umfasst.

12. Peptid nach Anspruch 11, wobei das Peptid dimer ist.

13. Peptid nach Anspruch 1, wobei das Peptid ausgewählt ist aus der Gruppe, die aus SEQ ID No. 4, 9, 12, 15, 17, 22, 31, 32, 40, 41, 42, 43, 45, 52, 54, 60, 62, 63, 71, 94, 96, 123, 124, 125, 133, 140, 142, 144, 150 und 154 besteht.

14. Peptid nach Anspruch 1, das die Sequenz YYGNYGVY einschließt.

15. Peptid nach Anspruch 1, wobei das Peptid ausgewählt ist aus der Gruppe, die aus SEQ ID No. 4, SEQ ID No. 17, SEQ ID No. 22 und SEQ ID No. 45 besteht.

16. Pharmazeutische Zusammensetzung umfassend einen pharmazeutisch akzeptablen Träger und eine therapeutisch wirksame Menge eines Peptids nach Anspruch 1.

17. Pharmazeutische Zusammensetzung nach Anspruch 16, wobei das Peptid ausgewählt ist aus der Gruppe, die aus SEQ ID No. 4, 9, 12, 15, 17, 22, 31, 32, 40, 41, 42, 43, 45, 52, 54, 60, 62, 63, 71, 94, 96, 123, 124, 125, 133, 140, 142, 144, 150 und 154 besteht.

18. Verwendung einer Zusammensetzung, die einen pharmazeutisch akzeptablen Träger und eine therapeutisch wirksame Menge eines Peptids nach Anspruch 1 umfasst, für die Herstellung eines Medikaments.

19. Verwendung einer Zusammensetzung, die einen pharmazeutisch akzeptablen Träger und eine therapeutisch wirksame Menge eines Peptids nach Anspruch 1 umfasst, für die Herstellung eines Medikaments zur Behandlung einer entzündlichen Erkrankung in einem Patienten.

20. Verwendung von Anspruch 19, wobei die entzündliche Erkrankung eine entzündliche Erkrankung des Gehirns ist.

21. Verwendung von Anspruch 19, wobei die entzündliche Erkrankung des Gehirns multiple Sklerose ist.

22. Verwendung einer Zusammensetzung, die ein Peptid nach Anspruch 1 umfasst, für die Herstellung eines Medikaments zum Inhibieren der durch VLA-4 vermittelten Adhäsion von Leukozyten.

## Revendications

1. Peptide possédant une affinité de liaison à VLA-4 telle qu'elle est exprimée par une IC₅₀ d'environ 50 µM ou moins, lequel peptide est choisi dans le groupe constitué par les peptides définis par la formule :
[R¹-Y/F-G/E-R²]ₙ
qui comprend une séquence d'acides aminés YYGN ;
et les peptides répondant à la formule :
R-PVSF-R'
où R et R' sont indépendamment des séquences peptidiques portant de 0 à 7 résidus d'acides aminés à condition que la somme des résidus d'acides aminés dans R + R' ne soit pas supérieure à 9,
R¹ est une séquence peptidique portant de 0 à 6 résidus d'acides aminés et R² est une séquence peptidique portant de 1 à 7 résidus d'acides aminés à condition que la somme des résidus d'acides aminés dans R¹ + R² soit comprise entre 2 et 11, et
n est un nombre entier égal soit à 1 soit à 2.

2. Peptide selon la revendication 1, dans lequel un ou plusieurs des résidus d'acides aminés sont des résidus d'acides aminés de la série D.

3. Peptide selon la revendication 1, dans lequel l'acide aminé N-terminal dudit résidu d'acide aminé est modifié par liaison de l'amine à un groupe R₄-CO- dans lequel R₄ est un atome d'hydrogène, un groupe alkyle de 1 à 10 atomes de carbone, un groupe cycloalkyle, aryle, arylalkyle.

4. Peptide selon la revendication 1, dans lequel l'acide aminé N-terminal dudit résidu d'acide aminé est modifié par liaison de l'amine à un groupe R₅O- dans lequel R₅ est un groupe alkyle de 1 à 10 atomes de carbone, un groupe cycloalkyle, aryle, arylalkyle.

5. Peptide selon la revendication 1, dans lequel l'acide aminé C-terminal dudit peptide est modifié par la liaison du carbonyle [C(O)] à un groupe répondant à la formule : 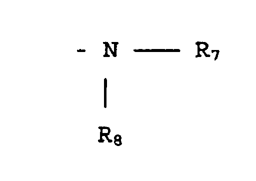 dans laquelle R₇ et R₈ sont identiques ou différents et sont choisis dans le groupe constitué par un atome d'hydrogène, un groupe alkyle de 1 à 10 atomes de carbone, un groupe cycloalkyle, aryle ou arylalkyle.

6. Peptide selon la revendication 1, dans lequel le résidu d'acide aminé C-terminal dudit peptide est lié à un groupe répondant à la formule -O-R₆ dans laquelle R₆ est choisi dans le groupe constitué par un groupe alkyle de 1 à 10 atomes de carbone, un groupe cycloalkyle, aryle ou arylalkyle.

7. Peptide selon la revendication 3, dans lequel le résidu d'acide aminé C-terminal dudit peptide est lié à un groupe répondant à la formule -O-R₆ dans laquelle R₆ est choisi dans le groupe constitué par un groupe alkyle de 1 à 10 atomes de carbone, un groupe cycloalkyle, aryle ou arylalkyle.

8. Peptide selon la revendication 4, dans lequel le résidu d'acide aminé C-terminal dudit peptide est lié à un groupe répondant à la formule -O-R₆ dans laquelle R₆ est choisi dans le groupe constitué par un groupe alkyle de 1 à 10 atomes de carbone, un groupe cycloalkyle, aryle ou arylalkyle.

9. Peptide selon la revendication 1, dans lequel le peptide est constitué d'environ 6 à environ 10 résidus d'acides aminés.

10. Peptide selon la revendication 1, dans lequel le peptide est constitué d'environ 8 à environ 10 résidus d'acides aminés.

11. Peptide selon la revendication 1, dans lequel le peptide comprend C sur l'acide aminé C-terminal.

12. Peptide selon la revendication 11, dans lequel le peptide est dimèrique.

13. Peptide selon la revendication 1, dans lequel le peptide est choisi dans le groupe constitué par les SEQ ID NO : 4, 9, 12, 15, 17, 22, 31, 32, 40, 41, 42, 43, 45, 52, 54, 60, 62, 63, 71, 94, 96, 123, 124, 125, 133, 140, 142, 144, 150 et 154.

14. Peptide selon la revendication 1, qui comprend la séquence YYGNYGVY.

15. Peptide selon la revendication 1, dans lequel le peptide est choisi dans le groupe constitué par les SEQ ID NO : 4, SEQ ID NO : 17, SEQ ID NO : 22 et SEQ ID NO : 45.

16. Composition pharmaceutique comprenant un véhicule pharmaceutiquement acceptable et une quantité thérapeutiquement efficace d'un peptide selon la revendication 1.

17. Composition pharmaceutique selon la revendication 16, dans laquelle le peptide est choisi dans le groupe constitué par les SEQ ID NO : 4, 9, 12, 15, 17, 22, 31, 32, 40, 41, 42, 43, 45, 52, 54, 60, 62, 63, 71, 94, 96, 123, 124, 125, 133, 140, 142, 144, 150 et 154.

18. Utilisation d'une composition comprenant un véhicule pharmaceutiquement acceptable et une quantité thérapeutiquement efficace d'un peptide selon la revendication 1 pour la fabrication d'un médicament.

19. Utilisation d'une composition comprenant un véhicule pharmaceutiquement acceptable et une quantité thérapeutiquement efficace d'un peptide selon la revendication 1 pour la fabrication d'un médicament pour le traitement d'une maladie inflammatoire chez un patient.

20. Utilisation selon la revendication 19, dans laquelle la maladie inflammatoire est une affection cérébrale inflammatoire.

21. Utilisation selon la revendication 19, dans laquelle l'affection cérébrale inflammatoire est une sclérose en plaques.

22. Utilisation d'une composition comprenant un peptide selon la revendication 1 pour la fabrication d'un médicament destiné à inhiber l'adhésion des leucocytes faisant intervenir comme médiateur VLA-4.
